# EUROPÄISCHE PATENTSCHRIFT

(11) **EP 3 638 324 B1**
(45) Veröffentlichungstag und Bekanntmachung des Hinweises auf die Patenterteilung: **30.04.2025**
(21) Anmeldenummer: 19708838.8
(22) Anmeldetag: 01.03.2019
(51) Int. Cl.: A61L 2/26, B65D 1/38, A61B 50/20

(54) **VERFAHREN ZUM HERSTELLEN EINER STERILISIERSIEBSCHALE MIT EINEM DREIDIMENSIONAL STRUKTURIERTEN BODEN**
METHOD FOR PRODUCING A STERILISABLE STRAINER DISH HAVING A THREE-DIMENSIONALLY STRUCTURED BOTTOM
PROCÉDÉ POUR LA FABRICATION D'UN PLATEAU PERFORÉ DE STÉRILISATION POURVU D'UN FOND STRUCTURÉ À TROIS DIMENSIONS

(30) Priorität: 05.03.2018 DE 102018104939
(43) Veröffentlichungstag der Anmeldung: 22.04.2020
(73) Patentinhaber: Aesculap AG, 78532 Tuttlingen (DE)
(72) Erfinder: GÖRZ, Dennis, 78532 Tuttlingen (DE); ROSIN, Bianca, 78532 Tuttlingen (DE); STREIT, Eva, 78351 Bodam-Ludwigshafen (DE); KNITTEL, Timo, 78573 Wurmlingen (DE)
(74) Vertreter: Winter, Brandl - Partnerschaft mbB
(86) Internationale Anmeldenummer: PCT/EP2019/055183
(87) Internationale Veröffentlichungsnummer: WO 2019/170551

(56) Entgegenhaltungen:
- DE-A1- 10 124 253
- DE-A1- 102010 050 919
- DE-U1- 202006 011 942
- US-A- 5 183 643

## Beschreibung

Die vorliegende Erfindung betrifft ein Verfahren zum Herstellen einer Sterilisiersiebschale, auch Siebkorb genannt, etwa einen Sterilisations- oder Desinfektionssiebkorb, zur Aufnahme zu desinfizierender oder sterilisierender medizinischer Gegenstände gemäß dem Oberbegriff des Anspruchs 1. Gattungsgemäße Siebkörbe werden eingesetzt, um in einem Reinigungs- und Desinfektionsgerät (RDG) oder Autoklaven in einer Aufbereitungseinheit für Medizinprodukte (AEMP) einen tragbaren Aufnahmebehälter für eine Anzahl zu desinfizierender oder sterilisierender Gegenstände, etwa chirurgische Greifinstrumente, bereitzustellen.

### Hintergrund der Erfindung

Ein Siebkorb hat primär die Aufgabe, in seinem Siebkorbinneren mehrere Gegenstände aufzunehmen, sodass diese in ihrem Reinigungsprozess als eine Einheit handhabbar sind. Ein Ver- oder Wegrutschen der aufgenommenen Gegenstände während des Packprozesses gilt es - im Sinne einer kompakten Beladung des Siebkorbs - zu vermeiden.

Weiterhin sollte ein Siebkorb möglichst wenig bis gar keine Abschnitte aufweisen, in denen sich während des Reinigungsprozesses eingesetzte Reinigungsflüssigkeit (d.h. mit Zusätzen versehenes Wasser) sammelt, damit die eingesetzte Reinigungsflüssigkeit nach dem Reinigungsprozess möglichst vollständig aus dem Siebkorb abläuft / abtropft.

Letztlich ist bei Siebkörben darauf zu achten, dass sie auch nach intensiver Benutzung, während der sie Stoß- und Schneidebelastungen ausgesetzt sind, keine für Brüche oder Risse anfälligen Abschnitte aufweisen, sodass das Verletzungs- und Schnittrisiko eines Bedieners minimiert ist.

### Stand der Technik

Aus der DE 20 2006 011 942 U1 ist ein Siebkorb bekannt, welcher zur Aufnahme zu desinfizierender oder sterilisierender Gegenstände angepasst ist. Der Siebkorb weist einen Boden, insbesondere einen Blechboden, auf, der mit einer Vielzahl von Durchbrechungen versehen ist. Der Boden hat eine von Seitenwänden begrenzte Grundebene, welche in einem Umformschritt hergestellt wird.

Diese Grundebene ist als eine Art Lochblech plan ausgestaltet. Um ein Ver- oder Wegrutschen der aufgenommenen Gegenstände zu vermeiden, wird in den Siebkorb eine Matte eingelegt. Diese Matte verhindert das effiziente Abtropfen des Siebkorbs, sodass Restwasser auch nach dem Entnehmen des Siebkorbs aus dem RDG eine störende Befeuchtung verschiedener Siebkorbbereiche, insbesondere des Siebkorbbodens, hervorruft.

Das Problem der störenden Befeuchtung des Siebkorbbodens liegt auch dann noch vor, wenn auf das Einlegen der Matte verzichtet wird. So bewirkt die plane Grundebene, dass zwischen den vom Siebkorb aufgenommenen Gegenständen und dem Boden entsprechend der verbleibenden Stegbreiten zwischen den Plattenlöchern ein Flächenkontakt ausgebildet ist, an dem sich unter Kapillarwirkung Restwasser sammelt, was wiederum die störende Befeuchtung fördert.

Eine weitere Gattung von Siebkörben aus dem Stand der Technik ist als Drahtgeflecht anstelle eines Lochblechs ausgestaltet. Ein Drahtgeflecht hat eine geriffelte Grundebene, die dem Ver- oder Wegrutschen der aufgenommenen Gegenstände entgegenwirkt. Weiterhin ruft ein Drahtgeflecht weniger Flächenkontakte beispielsweise mit einem planen Untergrund und folglich eine geringere Kapillarwirkung hervor.

Nachteilig an Siebkorb-Drahtgeflechten ist jedoch, dass sich in den einzelnen Knotenpunkten des Geflechts nicht nur Restwasser sondern auch Schmutz ansammelt. Weiterhin tritt in einem Drahtgeflecht nach entsprechender Nutzungsdauer zwangsläufig ein Aufbrechen oder Ablösen der einzelnen Drähte auf, was die Verletzungsgefahr, die von einem Siebkorb-Drahtgeflecht ausgeht, erheblich vergrößert.

Des Weiteren entspricht bei einem Siebkorb-Drahtgeflecht die Wandperforation der Bodenperforation. Wenn das Drahtgeflecht also ausreichend grob geflochten ist, um das Ver- oder Wegrutschen der aufgenommenen Gegenstände auf der Grundebene zu verhindern, hat es den Nachteil, dass die Wandperforation so grob ist, dass die aufgenommenen Gegenstände durch sie hindurchragen können, was erneut die Verletzungsgefahr erhöht und die Handhabung erschwert.

Letztlich ist ein Drahtgeflecht-Siebkorb auch aufwendiger in der Herstellung, da hier kein kostengünstiges Blech als Ausgangsmaterial zugrundeliegt.

Siebkörbe sind ebenfalls bekannt aus der DE 101 24 253 A1 und der DE 10 2010 050919 A1.

So weisen die Siebkörbe aus dem Stand der Technik und ihre Herstellungsverfahren allesamt den Nachteil auf, dass sie auch nach dem Entnehmen aus dem RDG von anhaftendem Restwasser hervorgerufen weitere Oberflächen störend befeuchten. Weiterhin haben sie je nach Ausgestaltung etwa den Nachteil der erhöhten Verletzungsgefahr, den Nachteil des Ver- oder Wegrutschens der aufgenommenen Gegenstände und/oder den Nachteil des Verschmutzens bzw. der Anreicherung von Keimen. Zudem sind ihre Herstellungsverfahren insgesamt wenig ausgereift.

### Kurzbeschreibung der Erfindung

Der vorliegenden Erfindung liegt angesichts dieses Standes der Technik die Aufgabe zugrunde, die Nachteile aus dem Stand der Technik zu beheben oder zumindest zu mildern, und insbesondere ein effizientes Herstellungsverfahren zu offenbaren, das die Massenproduktion von Siebkörben, die schnell abtropfen, kompakt beladbar sind und von denen ein geringes Verletzungsrisiko ausgeht, wirtschaftlich ermöglicht.

Dies wird erfindungsgemäß mittels eines Verfahrens mit den Merkmalen des Anspruchs 1 gelöst. Vorteilhafte Ausführungsformen sind Gegenstand der Unteransprüche.

Es hat sich gezeigt, dass das Ausbilden oder Anordnen von Abstandsfüßen/Noppen beispielsweise an der Außen-/Unterseite einer zu einem Siebkorbboden verarbeiteten planen Lochplatte ggf. die Kontaktfläche zwischen dem Siebkorb und einem planen Untergrund reduziert und damit das Abtropfverhalten insgesamt verbessern könnte, jedoch die Fertigung einer solchen speziellen Platte aufwändig und teuer und damit insgesamt unwirtschaftlich wäre. Darüber hinaus wären die Abstandsfüße/Noppen mit einem bestimmten Abstand zueinander über die Plattenfläche verteilt, welche sich daher je nach Wahl des Abstandes zwischen zwei benachbarten Abstandsfüßen/Noppen ggf. durchbiegen könnte. Aus diesen Gründen hat sich eine derartige Lösung des eingangs gestellten Problems als nicht zielführend erwiesen.

Der Grundgedanke der vorliegenden Erfindung verfolgt nunmehr das Grundkonzept, die räumliche Struktur eines Drahtgeflechtes bei einer zunächst planen Lochplatte zu simulieren, indem die zwischen den Plattenlöchern (Poren) sich erstreckenden (und sich kreuzenden) Stege zumindest teil- oder abschnittsweise in eine oder in mehrere Richtungen unterschiedlich zu deren jeweiliger Steg-Erstreckungsrichtung deformiert werden, wodurch die deformierten Stege selbst zumindest teilweise punktartige Kontaktbereiche beispielsweise mit einem planen Untergrund definieren. Dies erlaubt es, quasi jede zunächst plane Lochplatte mit dieser zusätzlichen Fähigkeit, nämlich der Bereitstellung einer nahezu beliebig wählbaren Anzahl von punktartigen Kontaktbereichen in Form von entsprechend dreidimensional sich erstreckender Plattenstege zwischen den Plattenlöchern auszubilden/nachzurüsten, etwa indem die zunächst plane Lochplatte einem entsprechenden (finalen) Deformationsschritt unterzogen wird.

Unter dem Begriff "Lochplatte" wird im Rahmen dieser Anmeldung eine sich in zwei Haupt-Raumrichtungen erstreckende Platte vorzugsweise aus Blechmaterial mit der konstanten Dicke / Materialstärke des Blechs in eine dritte Neben-Richtung verstanden. Durch die Ausbildung von Wellungen oder Ein-/Ausbeulungen insbesondere in die dritte Neben-Richtung erhält die Lochplatte zusätzlich eine dreidimensionale, variierende Struktur, die von jener planen Platte mit der konstanten Plattendicke/Plattenstärke abweicht. Vorteilhaft ist es dabei, die Ein-/Ausbeulungen nicht im Makrobereich auszubilden, d.h. eine einzige Ein-/Ausbeulung über eine Mehrzahl/Vielzahl von Stegen hinweg zu formen, sondern die Ein-/Ausbeulungen im Mikrobereich vorzusehen, d.h. die jeweilige Ein-/Ausbeulung beispielsweise im Wesentlichen innerhalb/längs jeweils eines Stegs (vorzugsweise zwischen zwei oder drei Kreuzungspunkten/Knoten mit den jeweils anderen Stegen) und/oder an jeweils einem ausgewählten Kontenpunkt (Bereich innerhalb einer Anzahl von kreisförmig einen einzigen Knotenpunkt als Mittel- und damit Kontaktpunkt unmittelbar umgebender Knotenpunkte) zu formen.

Aus diesem erfindungsgemäßen Verfahren zur Herstellung einer dreidimensionalen Lochplatte lassen sich beispielsweise folgende weitere Vorteile ableiten:
- Das Verfahren lässt sich automatisieren, wodurch die Produktionskosten, insbesondere bei den angestrebten Großserien, minimiert werden;
- Oberflächen, mit denen der erfindungsgemäß hergestellte Siebkorb nach der Entnahme aus dem RDG in Kontakt kommt, werden von diesem nicht bzw. deutlich weniger befeuchtet.
- Durch den Einsatz eines Blech-Rohlings kann auf ein Drahtgeflecht verzichtet werden, was die Herstellungskosten senkt und außerdem die Stichgefahr an dem hergestellten Siebkorb ausräumt.
- Die Perforationsgeometrie des Bodens lässt sich durch den Trenn- bzw. Stanzschritt unabhängig von der der Seitenwände gestalten, sodass diese jeweils an die unterschiedlichen Anforderungen angepasst werden.
- Ein Weg- oder Abrutschen der aufgenommenen Gegenstände lässt sich auch ohne das Einlegen einer (Silikon-)Matte verhindern.

Gemäß der Erfindung weist zumindest der Boden des Siebkorbs eine Lochplatte vorzugsweise als Blechteil auf oder besteht aus dieser, das, vorzugsweise periodische, dreidimensionale Wellungen oder Einbeulungen, auch Ein-/Ausbuchtungen oder Eindellungen, aufweist/erhält, die aus einer durch die zunächst plane Lochplatte gebildeten Grundebene zum Siebkorbinneren / nach innen und/oder zum Siebkorbäußeren / nach außen herausragen, sodass der Lochplatten- oder BlechBoden eine Oberfläche nach Art eines (Draht-)Geflechts aufweist/erhält. So wird von einer Lochplatte/Blechteil eine Geflechtoberflächenstruktur imitiert / simuliert / nach-oder abgebildet, wodurch die Struktur eines Geflechts und die eines Lochblechs synergistisch miteinander kombiniert werden. Bevorzugt hat/erhält zumindest das Blechteil bzw. die Lochplatte zumindest des Siebkorbbodens eine Anzahl von Durchgangslöchern (Poren), die von sich überschneidenden / kreuzenden Stegen umfangsseitig begrenzt und entsprechend der Stegbreite voneinander beabstandet sind. Erfindungsgemäß werden in/an einzelnen Stegen Deformationen (Ausbauchungen/Auszackungen) in eine oder in mehrere Richtungen unterschiedlich zu deren jeweiliger Steg-Erstreckungsrichtung insbesondere (wechselweise) in Richtung Außen und/oder Innenseite des Siebkorbs ausgebildet, wodurch eine raue/gezahnte Aufstandsfläche an der Bodenaußenseite und/oder eine raue/gezahnte Auflagefläche an der Bodeninnenseite entsteht.

Um eine solche dreidimensionale Lochplatte herzustellen, betrifft der Gegenstand der Erfindung demnach ein Verfahren zum Herstellen einer solchen Lochplatte und eines mit dieser Lochplatte versehenen Siebkorbs zur Aufnahme zu desinfizierender oder sterilisierender medizinischer Gegenstände. Bei dem Verfahren werden, basierend auf einem Platten- insbesondere Blech-Rohling folgende Schritte (nicht zwingend chronologisch) durchgeführt:
1 ein erster Bearbeitungsschritt, vorzugsweise ein Trennen, besonders bevorzugt ein Nibbeln oder beispielsweise auch ein Schneiden, wie ein Laser- oder ein Wasserstrahlschneiden, stellt aus dem Platten- bzw. Blech-Rohling eine plane Siebkorb-Grundfläche her bestehend aus dem (späteren) Siebkorbboden und den einstückig sowie plan sich daran anschließenden (späteren) Siebkorbseitenwänden;
2 ein (zeitlich vor oder nach dem ersten Bearbeitungsschritt ablaufender) zweiter Bearbeitungsschritt, vorzugsweise ein Trennen, besonders bevorzugt ein Stanzen, versieht den Blech-Rohling bzw. die Siebkorb-Grundfläche mit Durchbrechungen/Löchern/Poren, insbesondere im Bereich des (späteren) Siebkorbbodens und optional in ausgewählten/auswählbaren Bereichen der (späteren) Siebkorbseitenwände ggf. mit zum Siebkorbboden unterschiedlicher Lochgröße, um eine perforierte Ausgangsform zu erhalten;
3 anschließend ein zeitlich nach dem ersten und dem zweiten Bearbeitungsschritt ablaufender dritter Bearbeitungsschritt, vorzugsweise ein Umformen, besonders bevorzugt ein Walzen / "Glätten", stellt eine perforierte Ebene her. (Dieser Schritt ist notwendig, da es während des ersten und/oder des zweiten Bearbeitungsschrittes zu Verformungen/Verwölbungen innerhalb des Blechs kommt, die etwa von Eigenspannungen hervorgerufen sind);
4 ein (zeitlich nach dem dritten Bearbeitungsschritt ablaufender) vierter Bearbeitungsschritt, vorzugsweise ein Umformen, besonders bevorzugt ein Biegen/Abkanten des Seitenwandbereichs gegenüber dem Bodenbereich, stellt eine Siebkorb-(Grund-)Form her, deren Roh-Boden dem ebenen Innenabschnitt der perforierte Ebene und die Seitenwände den ebenen Außenabschnitten der perforierten Ebene entspricht/entsprechen.

So ist auf der Basis eines günstig zu beschaffenden und komfortabel durch Schneiden, Stanzen und Biegen zu bearbeitenden Blechs in wenigen Schritten eine Siebkorb-Grundform hergestellt, welche (nach Verschweißen der Seitenwände miteinander) theoretisch bereits geeignet wäre, in einem RDG eingesetzt zu werden.

Gemäß der Erfindung ist zeitlich nach dem dritten Bearbeitungsschritt, jedoch nicht zwingend nach dem vierten Bearbeitungsschritt ein fünfter Bearbeitungsschritt, vorzugsweise ein Umformen, besonders bevorzugt ein (Ein-)Prägen, vorgesehen, der zumindest aus dem ebenen Innenabschnitt (welcher den Siebkorbboden definiert) zumindest teilweise einen dreidimensional strukturierten Boden mit der vorstehend beschriebenen Struktur ergibt. Auf diese Weise ist der zuvor ebene Boden des Siebkorbs mit einer geriffelten Struktur versehen, ohne dass es während des fünften Bearbeitungsschritts / des Einprägens zu Verformungen in anderen, nicht zur Prägeverformung vorgesehenen Bereichen des Siebkorbs kommt.

In anderen Worten lässt sich die Erfindung funktional derart beschreiben, dass ein Siebkorb mit einem Boden aus einer Lochplatte vorzugsweise aus Blech hergestellt wird, welcher Boden die Oberflächen-Geometrie und Struktur eines Geflechts aufweist, ohne jedoch geflochten zu sein. So entfaltet die Erfindung die Wirkung, dass sie die Vorteile eines Geflechts (Bodenstruktur mit Anlage- und Fixierungsflächen, effizientes Abtropfen) ohne dessen Nachteile (s.u.) realisiert. Ein Flechten bringt mit sich, dass sich zwei verflochtene Stränge / Drähte in den Knotenpunkten überlappen / übereinanderliegen. Ein solches Überlappen ist für Siebkörbe mit dem erheblichen Nachteil verbunden, dass sich in der Überlappungsstelle, d.h. dem Knotenpunkt, Keime und Schmutzpartikel anreichern, da diese kaum zu erreichen und somit zu reinigen sind. Des Weiteren ist ein Geflecht deutlich aufwendiger in der Herstellung als eine Blechbearbeitung. So räumt die erfindungsgemäße "Geflecht-Simulation" die Nachteile aus dem Stand der Technik effizient aus.

Der erfinderische Gedanke liegt indessen darin, dass durch das Herstellen eines geflechtähnlichen / geflechtsimulierenden Boden-Blechs zum einen ein Wegrutschens der zu reinigenden Gegenstände während des Pack-Prozesses des Siebkorbs verhindert wird, während zum zweiten das Vorhandensein von Restwasser im Siebkorb nach dem Reinigungsprozess im RDG reduziert oder sogar vermieden ist, sodass es zu keiner störenden Befeuchtung (etwa des Packtischs) kommt.

In diesem Zusammenhang bleibt zu erwähnen, dass sich die in dieser Anmeldung beschriebenen Bearbeitungsschritte jeweils aus mehreren Teilschritten zusammensetzen können.

In einer vorteilhaften Ausführungsform der Erfindung läuft der fünfte Bearbeitungsschritt / das Einprägen zeitlich vor dem vierten Bearbeitungsschritt ab. In diesem Fall findet das Einprägen der dreidimensionalen Struktur unmittelbar nach dem Glätten der bereits in die Innen- und Außenbereiche geschnittenen und Lochgestanzen Grundplatte/Werkstücks statt, wodurch die so teilbearbeitete Grundplatte / das Werkstück während des Einprägens immer noch eine geringe Höhe aufweist (weil noch keine Seitenwände gebogen sind), sodass ein eingesetztes Einpräge-Werkzeug in der Höhenrichtung nur wenig zu bewegen ist. Zudem wäre es in diesem Fall möglich, auch die als Seitenwände vorgesehenen Außenbereiche der Grundplatte entsprechend zu prägen.

Alternativ zu dieser Ausführungsform können die Bearbeitungsschritte vom ersten Bearbeitungsschritt bis zum fünften Bearbeitungsschritt zeitlich chronologisch in genau dieser Reihenfolge ablaufen. So wird das geschnittene sowie bereits Loch-gestanzte Blechteil zum Siebkorb mit vom Boden senkrecht sich erstreckenden Seitenwänden gebogen, bevor der Boden (ein-)geprägt wird (fünfter Schritt), um die dreidimensionale Struktur gemäß vorstehender Definition hervorzurufen. Dieses Vorgehen hat dann den Vorteil, dass Herstellungsverfahren aus dem Stand der Technik dahingehend umrüstbar sind, dass im Anschluss an das bisherige Verfahren der erfindungsgemäß fünfte Bearbeitungsschritt durchzuführen ist bzw. bereits existierende Siebkörbe aus Lochblech mit einer entsprechenden dreidimensionalen Struktur nachträglich versehen werden können.

Bevorzugt läuft zeitlich nach dem vierten Bearbeitungsschritt ein sechster Bearbeitungsschritt, vorzugsweise ein (etwa stoffschlüssiges) Fügen, besonders bevorzugt ein Schweißen, wie ein Schmelzverbindungsschweißen, ab, welcher die einzelnen Randabschnitte, die nunmehr Seitenwände des Siebkorbs darstellen, randseitig miteinander fest verbindet. Der fünfte Bearbeitungsschritt ist weiter wahlweise vor oder nach dem sechsten Bearbeitungsschritt durchzuführen. Der sechste Bearbeitungsschritt garantiert eine robuste Konstruktion des Siebkorbs.

Eine weitere vorteilhafte Ausführungsform ist dadurch gekennzeichnet, dass die in dem zweiten Bearbeitungsschritt / dem Stanzen erhaltenen Durchbrechungen/Löcher in dem ebenen Innenabschnitt und dem Außen- oder Randabschnitt zueinander unterschiedlich strukturierte Perforierungen hervorrufen. So weist das Stanzwerkzeug für eine solche Strukturierung über die Grundfläche verteilt verschieden ausgestaltete oder auswählbare Wirkflächen auf.

Vorteilhafterweise wird bei dem fünften Bearbeitungsschritt / dem (Ein-)prägen zumindest ein Stempel, nach Art eines (Ein-)prägestempels, eingesetzt, welcher von einer Presse derart auf einen Teil zumindest des ebenen Innenabschnitts (nachfolgend Siebkorbboden) der bereits geschnittenen und gelochten Grundplatte gedrückt wird, dass sich der Teil des Innenabschnitts plastisch an die Negativform des Stempels anschmiegt. So lässt sich die erwünschte dreidimensionale Struktur mit hoher Präzision fertigen.

Weiter vorteilhaft wird in diesem Einprägeschritt eine Vielzahl von Stempeln eingesetzt, sodass der gesamte ebene Innenabschnitt als dreidimensional strukturierter Boden ausgestaltet ist. Weiter bevorzugt haben die entsprechenden Stempel dieselbe Struktur, sodass der ebene Innenabschnitt einheitlich verformt ist. Dadurch, dass der gesamte Innenabschnitt in dieser Ausführungsform als dreidimensional strukturierter Boden ausgestaltet ist, läuft das (eingangs erwähnte) Abtropfen des Siebkorbs schnell ab und ruft die damit einhergehenden Vorteile hervor.

In einer bevorzugten Ausführungsform weist der Stempel eine solche Form / Gestalt auf, dass der in dem fünften Bearbeitungsschritt hergestellte dreidimensional strukturierte Boden (periodische) Wellungen oder Einbeulungen aufweist, die zum Siebkorbinneren und/oder dem Siebkorbäußeren herausragen, sodass der Boden eine Oberfläche nach Art eines Geflechts aufweist. Diese Geflecht-Simulation verbindet die Vorteile von Blech-Siebkörben mit Draht-Siebkörben in synergistischer Art und Weise.

Eine weitere vorteilhafte Ausführungsform zeichnet sich dadurch aus, dass der Stempel dergestalt ist, dass der in dem fünften Bearbeitungsschritt hergestellte dreidimensional strukturierte Boden aus einer Vielzahl von in der Grundebene jeweils parallel verlaufenden Längsstrebenpaaren / Längsstegpaaren und Querstrebenpaaren / Querstegpaaren aufgebaut ist, die in einer Draufsicht auf den strukturierten Boden senkrecht zueinander verlaufen. Somit verformt der fünfte Bearbeitungsschritt den Boden ausschließlich in der Siebkorbhöhenrichtung, das heißt zum Siebkorbinneren und/oder dem Siebkorbäußeren, während eine für das Bepacken vorteilhafte Gitterstruktur in der Draufsicht erhalten bleibt.

Der in dem erfindungsgemäßen Verfahren eingesetzte Stempel ist weiter bevorzugt dergestalt, dass der dreidimensional strukturierte Boden Anlage- und Fixierungsflächen für in den Siebkorb einzulegende Gegenstände ausbildet. Auf diese Weise ist die die Lagestabilität der aufgenommenen Gegenstände erhöht und es ist keine (Silikon-)Matte auf den Boden zu legen, um ein sicheres, formschlüssiges Haften der einzelnen Gegenstände zu gewährleisten.

Die Erfindung wird nachstehend anhand bevorzugter Ausführungsbeispiele unter Bezugnahme auf die begleitenden Figuren näher erläutert. Die Figuren sind lediglich schematischer Natur und dienen ausschließlich dem Verständnis der Erfindung. Die gleichen Elemente sind mit denselben Bezugszeichen versehen. Es zeigen:
- Fig. 1:: ein Ablaufdiagramm des erfindungsgemäßen Herstellungsverfahrens;
- Fig. 2:: ein weiteres Ablaufdiagramm des erfindungsgemäßen Herstellungsverfahrens;
- Fig. 3:: einen Blechrohling, der dem Herstellungsverfahren zugrunde liegt;
- Fig. 4:: eine Siebkorb-Ausgangsform, die aus dem Blechrohling aus Fig. 3 hergestellt wird;
- Fig. 5: eine perforierte Ausgangsform (wenn noch kein Walzen stattgefunden hat) bzw. eine perforierte Ebene (wenn das Walzen bereits stattgefunden hat);
- Fig. 6: eine Siebkorb-Form nach einem Umbiegen;
- Fig. 7:: eine perspektivische Ansicht eines Siebkorbs;
- Fig. 8:: den mit viii angedeuteten Bereich aus Fig. 7 schematisch vergrößert;
- Fig. 9:: den mit ix angedeuteten Bereich aus Fig. 7 schematisch vergrößert;
- Fig. 10: eine perspektivische Ansicht eines Geflecht-imitierten Bodens;
- Fig. 11: eine perspektivische Ansicht einer weiteren Ausführungsform des Geflecht-imitierten Bodens; und
- Fig. 12: einen Ausschnitt des Siebkorbs mit eingelegten Gegenständen.

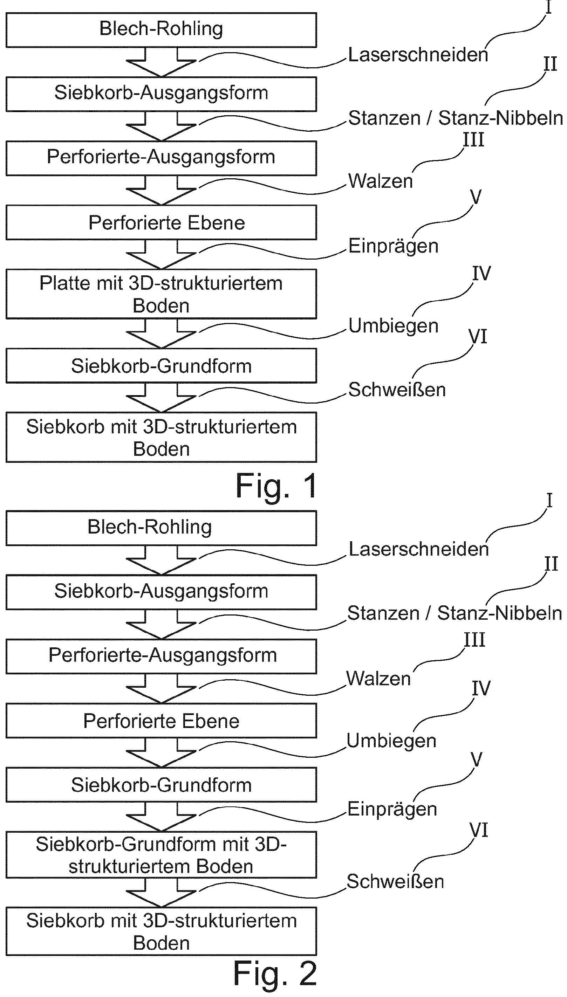

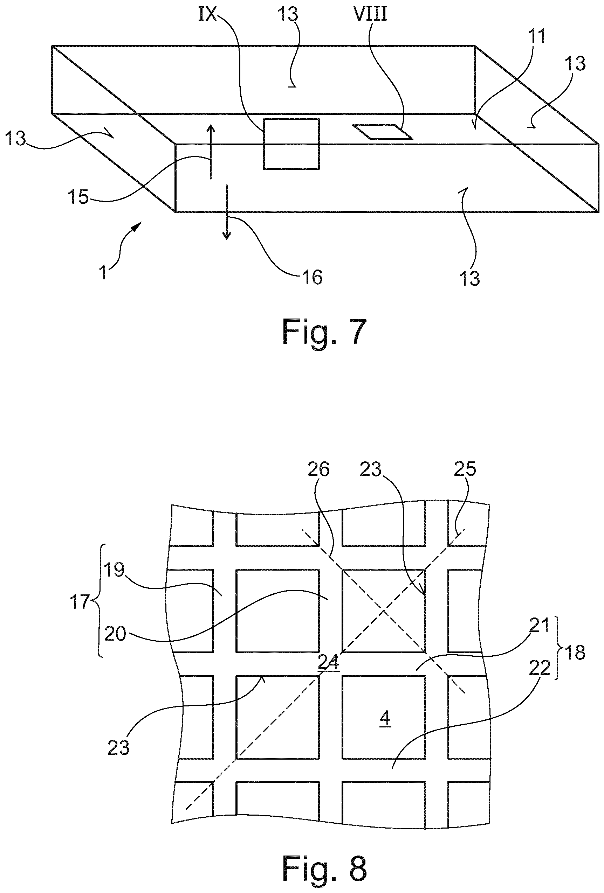

Fig. 1 zeigt eine erste Möglichkeit eines Verfahrensablaufs zur Herstellung eines Siebkorbs 1 (vgl. Fig. 7) chronologisch. Hierbei laufen ein Laserschneiden I, ein Stanzen II (auch Stanz-Nibbeln, sofern der auszustanzende Teil nur teilweise gestanzt und teilweise gebrochen wird), ein Walzen III, ein Einprägen V, ein Umbiegen IV und ein Schweißen VI chronologisch nacheinander ab.

Fig. 2 zeigt eine zweite Möglichkeit eines Verfahrensablaufs chronologisch. Hierbei laufen ein Laserschneiden I, ein Stanzen II (auch Stanz-Nibbeln, sofern der auszustanzende Teil nur teilweise gestanzt und teilweise gebrochen wird), ein Walzen III, ein Umbiegen IV, ein Einprägen V und ein Schweißen VI chronologisch nacheinander ab.

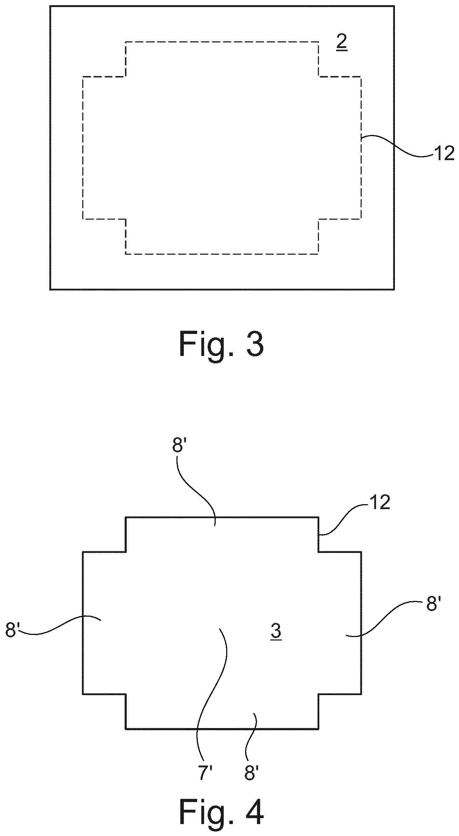

Die sich nach jedem Schritt ergebenden Bauteile, die bereits in den Kästen der Figuren 1 und 2 genannt sind, werden nun im Zusammenhang mit den Figuren 3 bis 7 näher erläutert.

In Fig. 3 ist ein rechteckiger Blech-Rohling 2 dargestellt. Dieser kann jede beliebige Form aufweisen. In seiner Materialstärke weist er rund 0,5 mm bis 2 mm, vorzugsweise rund 1,5 mm auf. Fig. 3 deutet bereits eine Schneidkontur 12 an, entlang derer das Laserschneiden I durchgeführt wird.

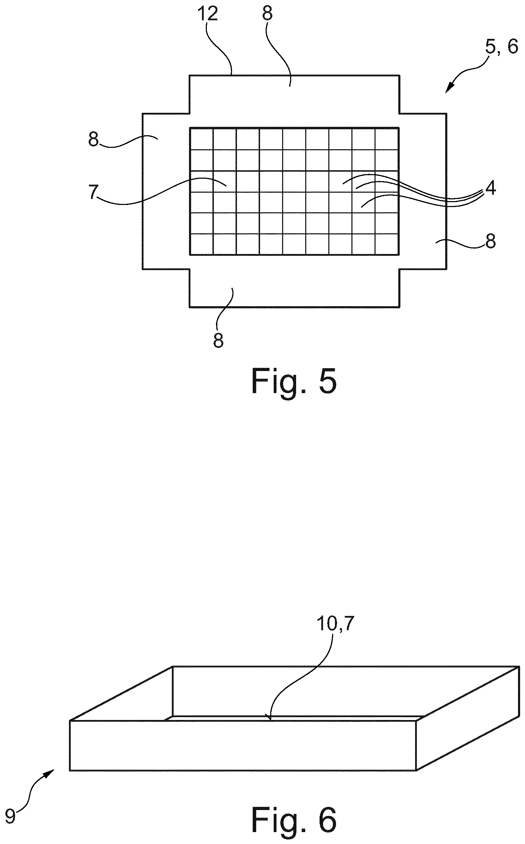

Fig. 4 zeigt eine Siebkorb-Grundfläche 3, die aus dem Blech-Rohling 2 entlang der Schneidkontur 12 ausgeschnitten wurde. Die Siebkorb-Grundfläche 3 weist bereits Bereiche 7' und 8' auf, welche nach weiterer Bearbeitung zu einem ebenen Innenabschnitt 7 bzw. Randabschnitten 8 modifiziert werden (vgl. Fig. 5).

Nach einem Stanz- bzw. Stanz-Nibbel-Schritt liegt eine perforierte Ausgangsform 5, vgl. Fig. 5, vor. Diese weist gestanzte Durchbrechungen 4 auf, wie sie in Fig. 5 schematisch angedeutet sind. Das Blech weist nach dem Schneiden I und dem Stanzen II in der Praxis gewisse Eigenspannungen auf, welche zu einer Verformung der Ausgangsform 5 führen. Folglich ist nunmehr das Walzen III durchzuführen, welche das Blech eben walzt / glättet, um so eine perforierte Ebene 6 zu erhalten. In der Draufsicht lässt sich die Ausgangsform 5 nicht von der perforierten Ebene 6 unterscheiden, weshalb in Fig. 5 beide Bezugszeichen verwendet wurden.

Des Weiteren lässt Fig. 5 den Innenabschnitt 7 und die Randabschnitte 8 erkennen. Diese werden vorzugsweise mit unterschiedlichen Werkzeugen gestanzt, sodass sie eine unterschiedliche Perforierung aufweisen.

Gegenstand des erfindungsgemäßen Einprägens V, das die gewünschte dreidimensionale Struktur nach Art eines Geflecht-Imitats hervorruft, ist ausschließlich der Innenabschnitt 7 oder der Innenabschnitt 7 und die Randabschnitte 8.

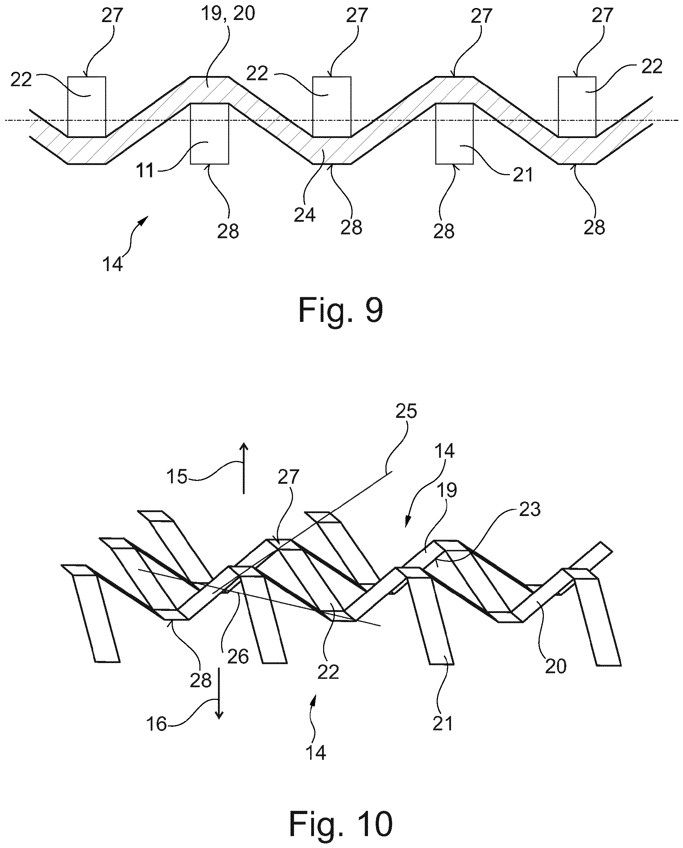

Nach dem Umbiegen IV erhält man eine Siebkorb-Form 9 (vgl. Fig. 6). Ein Roh-Boden 10 entspricht dem Innenabschnitt 7. Sobald das Einprägen V durchgeführt wurde, weist der (ebene) Roh-Boden 10 die erfindungsgemäßen Einwellungen (vgl. Fign. 9, 10) auf.

Fig. 7 zeigt einen Siebkorb 1 zur Aufnahme zu reinigender Gegenstände, mit einer Vielzahl von Durchbrechungen 4, wie sie auch in der Detailansicht in Fig. 8 erkennbar sind. Der vorliegend mit einer rechteckigen Grundfläche / Grundebene ausgebildete Siebkorb 1 weist einen dreidimensional strukturierten Boden 11 auf, von dem von jeder Seitenkante, vorliegend also vier, Seitenwände 13 abgehen.

Der Boden 11 weist, wie in der Detailansicht aus Fig. 9 erkennbar, periodische Wellungen bzw. Einbeulungen 14 auf. Diese Wellungen 14 ragen aus der Grundebene zum Siebkorbinneren 15 und vorliegend außerdem zum Siebkorbäußeren 16 heraus, sodass das Blechteil / der Boden 11 die Geflechtstrukturoberfläche annimmt.

Gemäß der Detailansicht aus Fig. 8, welche eine Draufsicht auf einen Ausschnitt des Bodens 11 darstellt, weist der Boden 11 in der Projektion eine Gitterstruktur auf. Diese setzt sich aus einer Vielzahl von in der Grundebene, d.h. in der vorliegenden Draufsicht, jeweils parallel verlaufenden Längsstrebenpaaren 17 (auch Längsstegpaar genannt) und Querstrebenpaaren 18 (auch Querstegpaar genannt) zusammen.

Ein einzelnes Längsstrebenpaar 17 setzt sich aus zwei Längsstreben 19, 20 zusammen. Diese Längsstreben 19, 20 verlaufen in der Grundebene, d.h. in der vorliegenden Draufsicht, zueinander parallel. Bei räumlicher Betrachtung (vgl. Fign. 9 und 10) ist erkennbar, dass jede Strebe 19, 20 in der dritten Raumrichtung, nämlich zum Siebkorbinneren 15 und/oder zum Siebkorbäußeren 16 hin, eine zueinander unterschiedliche, etwa komplementäre Geometrie aufweist.

Ein einzelnes Querstrebenpaar 18 setzt sich aus zwei Querstreben 21, 22 zusammen. Diese Querstreben 21, 22 verlaufen in der Grundebene, d.h. in der vorliegenden Draufsicht, zueinander parallel. Bei räumlicher Betrachtung (vgl. Fign. 9 und 10) ist erkennbar, dass jede Strebe 21, 22 in der dritten Raumrichtung, nämlich zum Siebkorbinneren 15 und/oder zum Siebkorbäußeren 16 hin, eine zueinander unterschiedliche, etwa komplementäre Geometrie aufweist.

Die von den Durchbrechungen 4 aufgespannte Fläche erfüllt zweierlei Funktionen. Zum ersten gibt sie an der zur Durchbrechung 4 hin gewandten Kantenfläche jeder Strebe 19 bis 22 eine Anlage- und Fixierungsfläche 23 frei. Diese Fläche 23 steigt mit der Größe der Durchbrechungen 4. Je größer die einzulegenden Gegenstände, desto größer sind folglich die Durchbrechungen 4 auszugestalten, damit ausreichend Anlage- und Fixierungsfläche 23 freigegeben ist. Zum zweiten ermöglicht die von den Durchbrechungen 4 aufgespannte Fläche ein Abtropfen der Reinigungsflüssigkeit aus dem Siebkorb 1 heraus. Auch wächst die Abtropffunktion mit der Größe der Durchbrechungen 4. Demnach regt auch jene zweite Funktion dazu an, das Flächenverhältnis zwischen den Strebenpaaren 17, 18 und der von den Durchbrechungen 4 aufgespannten Fläche kleiner 1 zu halten. Ein Maximum ist der von den Durchbrechungen 4 aufgespannten Fläche darin gesetzt, dass sie klein genug sein muss, um ein Herausfallen zu reinigender Geräte zu verhindern.

Die von dem Boden 11 definierte Gitterstruktur weist dieaus dem Einprägen V ergebene Bodenknoten 24 auf. Gemäß der Erfindung liegen diese Bodenknoten 24 nicht in derselben Ebene, da die Wellungen 14 ausgebildet sind. Ein besonderer Vorteil der Erfindung entfaltet sich dadurch, dass die Bodenknoten 24, die jeweils durch das Schneiden einer Längsstrebe 19, 20 mit einer Querstrebe 21, 22 gebildet sind, nahezu dieselbe Materialstärke aufweisen, wie die jeweilige Längs- oder Querstrebe 19 bis 22.

So ermöglicht die erfindungsgemäße Geflecht-Simulation nicht nur eine Nachahmung eines Geflechts, sondern weist gegenüber einem Geflecht noch den Vorteil auf, dass im Bereich des Knotens 24 keine Überlappung, sprich Verdoppelung der Materialstärke, vorliegt, sondern dieselbe konstante Materialstärke, wie im restlichen Boden. Bevor dieses Merkmal im Zusammenhang mit Fig. 9 weiter behandelt wird, seien noch zwei weitere Kenngrößen der vorliegenden Erfindung eingeführt.

So lässt sich ein Teil der Bodenknoten 24 miteinander hypothetisch verbinden, um die erste hypothetische Verbindungslinie 25 zu erkennen. Wie im weiteren Verlauf erkennbar ist, stellen die von der ersten hypothetischen Verbindungslinie 25 verbundenen Bodenknoten 24 solche Bodenknoten 24 dar, die gemäß einer vorteilhaften Ausgestaltung der Erfindung jeweils auf derselben Höhe angeordnet sind und ins Siebkorbinnere 15 hineinragen. Sie stellen sozusagen jeweils einen Wellenberg 27 (siehe hierfür Fig. 9) der periodischen Wellungen 14 dar.

In der Grundebene um 90° gedreht ist neben der Linie 25 eine zweite hypothetische Verbindungslinie 26 zu erkennen. Diese ergibt sich, wenn man die von der ersten hypothetischen Verbindungslinie 25 ausgelassenen Bodenknoten 24 miteinander verbindet. Wie im weiteren Verlauf erkennbar ist, stellen die von der zweiten hypothetischen Verbindungslinie 26 verbundenen Bodenknoten 24 solche Bodenknoten 24 dar, die gemäß einer vorteilhaften Ausgestaltung der Erfindung jeweils auf derselben Höhe angeordnet sind und zum Siebkorbäußeren 16 herausragen. Sie stellen sozusagen jeweils ein Wellental 28 (siehe hierfür Fig. 9) der periodischen Wellungen 14 dar.

Jene Wellenberge 27 und Wellentäler 28 sind in Fig. 9 dargestellt. Fig. 9 stellt eine Schnittzeichnung durch den Boden 11 dar (vgl. Bereich ix aus Fig. 7). Die hier gezeigte Strebe stellt eine Längsstrebe 19, 20 dar, welche sich strukturell jedoch in ihrer grundsätzlichen Form nicht von einer Querstrebe 21, 22 unterscheidet. Anhand der Sichtkanten lässt sich erkennen, dass von jedem Wellenberg 27 der Längsstrebe 19, 20 eine erste Querstrebe 21 abgeht, während von jedem Wellental 28 eine zweite Querstrebe 22 abgeht. Die vorstehend beschriebene Wechselseitigkeit der Wellenberge 27 und Wellentäler 28 lässt sich hier gut erkennen.

Die Längsstrebe 19, 20 weist vorliegend einen eckigen Verlauf auf. Diese Form hat jedoch nur beispielhaften Charakter. In anderen Ausführungsformen ist insbesondere eine annähernd Sinus-förmige Wellenform angestrebt.

Fig. 9 zeigt des Weiteren, dass die Materialstärke des Bodenknotens 24 die der restlichen Längsstrebe 19, 20 nicht übersteigt, wodurch es trotz der Geflecht-Simulation nicht zu einer unvorteilhaften Überlappung der Streben kommt, wie es eingangs beschrieben wurde.

In Fig. 10 sind die Wellungen 14 perspektivisch dargestellt. Die erste hypothetische Verbindungslinie 25 (vgl. Fig. 8) verbindet die Wellenberge 27, die zweite hypothetische Verbindungslinie 26 (vgl. Fig. 8) verbindet die Wellentäler 28. Die zwischen vier angrenzenden Bodenknoten 24 ausgebildete, dreidimensionale Dachform setzt sich aus zwei Dreiecken zusammen. Der Scheitel jener Dreiecke lässt sich, je nach Betrachtung, zwischen den zwei Wellenbergen 27 der vier angrenzenden Bodenknoten 24 ansetzen (dann ist das Dach zum Siebkorbinneren 15 hin geschlossen) oder zwischen den zwei Wellentälern 28 der vier angrenzenden Bodenknoten 24 ansetzen (dann ist das Dach zum Siebkorbinneren 15 hin offen und zum Siebkorbäußeren 16 hin geschlossen).

Fig. 10 lässt erkennen, dass die von dem Einprägen V geformte Struktur, welche in der projizierten Draufsicht aus Fig. 8 eine rechteckige Gitterstruktur zeigt, perspektivisch betrachtet ein hohes Maß an Räumlichkeit aufweist, welche das Benetzen jener Fläche von Tropfen nach dem Entnehmen aus dem RDG verringert. Zudem liefert die von den Wellungen 14 hervorgerufene Struktur ausreichend große Anlage- und Fixierungsflächen 23.

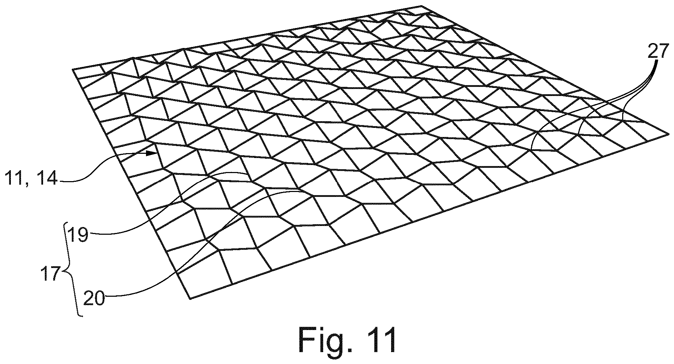

Fig. 11 zeigt die dreidimensionalen Wellungen 14 sowie die von ihnen hervorgerufene Riffelung in einem weiteren Abschnitt. Über die gesamte Fläche des Bodens 11 sind so viele Wellungen 14 angeordnet, dass die Gesamtheit an Wellenbergen 27 und Wellentälern 28 wiederum bei einem Anwender den Eindruck einer annährend planen Fläche entstehen lässt. So sind erfindungsgemäß die Vorteile einer planen Fläche (wie das problemlose Abstellen des Siebkorbs) unter Vermeidung von dessen Nachteilen (s.o.) verwirklicht.

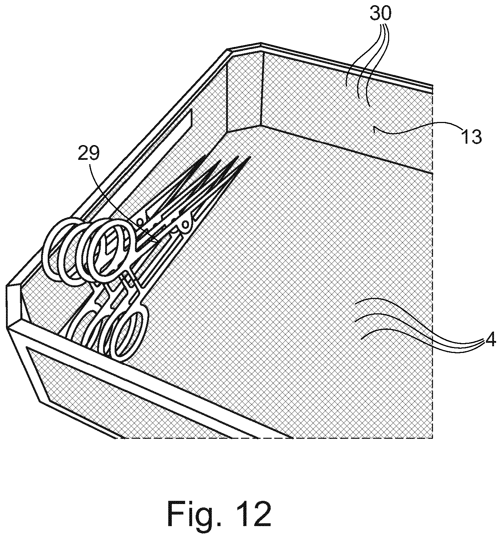

Fig. 12 stellt einen Abschnitt eines Siebkorbs 1 dar. Darin sind mehrere Gegenstände 29, vorliegend chirurgische Scheren, angeordnet, welche aufgrund der Wellungen 14 und der von ihnen hervorgerufenen Anlage- und Fixierungsflächen 23 in ihrer Position bleiben. Der Siebkorb 1 weist neben dem Boden 11 die Seitenwände 13 auf. Diese haben ebenso Durchbrechungen 30, welche sich geometrisch jedoch von denen im Boden 11 unterscheiden. Vorliegend sind die Durchbrechungen 30 feinmaschiger als die Durchbrechungen 4, damit in einem solchen Fall, in dem die Gegenstände 29 hin zur Seitenwand 13 rutschen, kein seitliches Herausragen von spitzen Abschnitten der Gegenstände 29 droht. Die Seitenwände 13 sind überdies ggf. glatt, d.h. explizit nicht gewellt ausgestaltet.

### Bezugszeichenliste

- 1: Siebkorb
- 2: Blech-Rohling
- 3: Siebkorb-Grundfläche
- 4: Durchbrechung
- 5: Grundform
- 6: perforierte Ebene
- 7: ebener Innenabschnitt
- 8: Randabschnitt
- 9: Siebkorb-Form
- 10: Roh-Boden
- 11: dreidimensional strukturierter Boden
- 12: Schneidkontur
- 13: Seitenwand
- 14: Wellungen bzw. Einbeulungen
- 15: Siebkorbinneres
- 16: Siebkorbäußeres
- 17: Längsstrebenpaar
- 18: Querstrebenpaar
- 19: Erste Längsstrebe
- 20: Zweite Längsstrebe
- 21: Erste Querstrebe
- 22: Zweite Querstrebe
- 23: Anlage- und Fixierungsfläche
- 24: Bodenknoten
- 25: Erste hypothetische Verbindungslinie
- 26: Zweite hypothetische Verbindungslinie
- 27: Wellenberg
- 28: Wellental
- 29: Gegenstand
- 30: Durchbrechungen der Seitenwand

- I: Erster Bearbeitungsschritt / Laserschneiden
- II: Zweiter Bearbeitungsschritt / Stanzen
- III: Dritter Bearbeitungsschritt / Walzen
- IV: Vierter Bearbeitungsschritt / Biegen
- V: Fünfter Bearbeitungsschritt / Einprägen
- VI: Sechster Bearbeitungsschritt / Schweißen

## Patentansprüche

1. Verfahren zum Herstellen eines Siebkorbs (1) zur Aufnahme zu desinfizierender oder sterilisierender medizinischer Gegenstände, bei dem aus einem Platten-, vorzugsweise Blech-Rohling (2)
- in einem ersten Bearbeitungsschritt (I), vorzugsweise einem Trennen, besonders bevorzugt einem Schneiden, wie einem Laserschneiden, eine den Boden und die Seitenwände des Siebkorbs einstückig umfassende Grundplatte oder Siebkorb-Grundfläche (3) hergestellt wird,
- in einem zeitlich vor oder nach dem ersten Bearbeitungsschritt (I) ablaufenden zweiten Bearbeitungsschritt (II), vorzugsweise einem Trennen, besonders bevorzugt einem Stanzen, der Platten- insbesondere Blech-Rohling (2) oder die Siebkorb-Grundfläche (3) mit Durchbrechungen oder Löchern (4) versehen wird, um eine perforierte Ausgangsform (5) zu erhalten,
- in einem zeitlich nach dem ersten und dem zweiten Bearbeitungsschritt (I, II) ablaufenden dritten Bearbeitungsschritt (III), vorzugsweise einem Umformen, besonders bevorzugt einem Walzen oder Plätten, eine perforierte oder gelochte Ebene (6) hergestellt wird, die in einen ebenen, den Boden repräsentierenden Innenabschnitt (7) und die Seitenwände repräsentierende Randabschnitte (8) eingeteilt ist,
- in einem zeitlich nach dem dritten Bearbeitungsschritt (III) ablaufenden vierten Bearbeitungsschritt (IV), vorzugsweise einem Umformen, besonders bevorzugt einem Biegen, eine Siebkorb-Form (9) mit Roh-Boden und vertikal hierzu sich erstreckender Seitenwände hergestellt wird, deren Roh-Boden (10) dem ebenen Innenabschnitt (7) und die Seitenwände den ebenen Randabschnitten (8) der perforierte Ebene (6) entsprechen,
**gekennzeichnet durch**
einen zeitlich unmittelbar oder mittelbar nach dem dritten Bearbeitungsschritt (III) ablaufenden fünften Bearbeitungsschritt (V), vorzugsweise ein Umformen, besonders bevorzugt ein Prägen, der zumindest aus dem ebenen Innenabschnitt (7) zumindest teilweise einen dreidimensional strukturierten Boden (11) herstellt.

2. Verfahren nach Anspruch 1, **dadurch gekennzeichnet, dass** der fünfte Bearbeitungsschritt (V) zeitlich vor dem vierten Bearbeitungsschritt (IV) abläuft.

3. Verfahren nach Anspruch 1, **dadurch gekennzeichnet, dass** die Bearbeitungsschritte vom ersten Bearbeitungsschritt (I) bis zum fünften Bearbeitungsschritt (V) zeitlich chronologisch in dieser Reihenfolge ablaufen.

4. Verfahren nach einem der vorstehenden Ansprüche, **dadurch gekennzeichnet, dass** zeitlich nach dem vierten Bearbeitungsschritt ein sechster Bearbeitungsschritt, vorzugsweise ein Fügen, besonders bevorzugt ein Schweißen, wie ein Schmelzverbindungsschweißen, abläuft, der die einzelnen Randabschnitte (8), welche nunmehr Seitenwände (13) des Siebkorbs (1) darstellen, miteinander verbindet.

5. Verfahren nach einem der vorstehenden Ansprüche, **dadurch gekennzeichnet, dass** die in dem zweiten Bearbeitungsschritt (II) erhaltenen Durchbrechungen (4) in dem ebenen Innenabschnitt (7) und dem Randabschnitt (8) zueinander unterschiedlich strukturierte Perforierungen hervorrufen.

6. Verfahren nach einem der vorstehenden Ansprüche, **dadurch gekennzeichnet, dass** bei dem fünften Bearbeitungsschritt (V) zumindest ein Stempel eingesetzt wird, welcher von einer Presse derart auf einen Teil des ebenen Innenabschnitts (7) gedrückt wird, dass sich der Teils des Innenabschnitts (7) plastisch an die Negativform des Stempels anschmiegt.

7. Verfahren nach Anspruch 6, **dadurch gekennzeichnet, dass** eine Vielzahl von Stempeln eingesetzt wird, sodass der gesamte ebene Innenabschnitt (7) als dreidimensional strukturierter Boden (12) ausgestaltet ist.

8. Verfahren nach einem der Ansprüche 6 oder 7, **dadurch gekennzeichnet, dass** der Stempel dergestalt ist, dass der in dem fünften Bearbeitungsschritt (V) hergestellte dreidimensionale strukturierte Boden (11) Wellungen oder Einbeulungen (14) aufweist, die zum Siebkorbinneren (15) und/oder Siebkorbäußeren (16) herausragen, sodass der Boden (11) eine Oberfläche nach Art eines Geflechts aufweist.

9. Verfahren nach einem der Ansprüche 6 bis 8, **dadurch gekennzeichnet, dass** der Stempel dergestalt ist, dass der in dem fünften Bearbeitungsschritt (V) hergestellte dreidimensionale strukturierte Boden (11) aus einer Vielzahl von in der Grundebene jeweils parallel verlaufenden Längsstrebenpaaren (17) und Querstrebenpaaren (18) aufgebaut ist, die in einer Draufsicht auf den strukturierten Boden (11) senkrecht zueinander verlaufen.

10. Verfahren nach einem der Ansprüche 6 bis 9, **dadurch gekennzeichnet, dass** der Stempel dergestalt ist, dass der dreidimensional strukturierte Boden (11) Anlage- und Fixierungsflächen (23) für in den Siebkorb (1) einzulegende Gegenstände (30) ausbildet.

## Claims

1. Method for producing a sieve basket (1) for receiving medical items to be disinfected or sterilized, in which from a plate blank (2), preferably a sheet metal blank (2),
- in a first processing step (I), preferably separating, particularly preferably cutting, such as laser cutting, a base plate or sieve basket base surface (3) comprising the bottom and side walls of the sieve basket in one piece is produced,
- in a second processing step (II) taking place prior to or after the first processing step (I), preferably separating, especially preferably punching, the plate blank, in particular sheet metal blank (2), or the sieve basket base surface (3) is provided with apertures or holes (4) in order to obtain a perforated initial shape (5),
- in a third processing step (III) taking place after the first and second processing steps (I, II), preferably forming, especially preferably rolling or flattening, a perforated or punched plane (6) is produced, which is divided into a flat inner portion (7) representing the bottom and edge portions (8) representing the side walls,
- in a fourth processing step (IV) taking place after the third processing step (III), preferably forming, especially preferably bending, a sieve basket shape (9) with a raw bottom and side walls extending vertically thereto is produced, the raw bottom (10) of which corresponds to the flat inner portion (7) and the side walls correspond to the flat edge portions (8) of the perforated plane (6),
**characterized by**
a fifth processing step (V) which takes place directly or indirectly after the third processing step (III), preferably forming, especially preferably embossing, which at least partially produces a three-dimensionally structured bottom (11) at least from the flat inner portion (7).

2. Method according to claim 1, **characterized in that** the fifth processing step (V) takes place prior to the fourth processing step (IV).

3. Method according to claim 1, **characterized in that** the processing steps from the first processing step (I) to the fifth processing step (V) run chronologically in this order.

4. Method according to one of the preceding claims, **characterized in that** after the fourth processing step, a sixth processing step, preferably joining, especially preferably welding, such as fusion joint welding, takes place, which connects the individual edge portions (8) together, which now constitute side walls (13) of the sieve basket (1).

5. Method according to one of the preceding claims, **characterized in that** the apertures (4) obtained in the second processing step (II) cause perforations which are structured differently from each other in the flat inner portion (7) and the edge portion (8).

6. Method according to one of the preceding claims, **characterized in that** in the fifth processing step (V), at least one punch is used, which is pressed by a press onto a part of the flat inner portion (7) in such a way that the part of the inner portion (7) plastically adapts to the negative shape of the punch.

7. Method according to claim 6, **characterized in that** a plurality of punches is used so that the entire flat inner portion (7) is designed as a three-dimensionally structured bottom (12).

8. Method according to one of claims 6 or 7, **characterized in that** the punch is designed in such a way that the three-dimensionally structured bottom (11) produced in the fifth processing step (V) has corrugations or indentations (14) which project towards the sieve basket interior (15) and/or towards the sieve basket exterior (16), so that the bottom (11) has a surface in the manner of a meshwork.

9. Method according to one of claims 6 to 8, **characterized in that** the punch is designed in such a way that the three-dimensionally structured bottom (11) produced in the fifth processing step (V) is composed of a plurality of, in the base plane parallel, longitudinal strut pairs (17) and transverse strut pairs (18), which run perpendicular to each other in a top view of the structured bottom (11).

10. Method according to one of claims 6 to 9, **characterized in that** the punch is designed in such a way that the three-dimensionally structured bottom (11) forms contact and fixing surfaces (23) for items (30) to be inserted into the sieve basket (1).

## Revendications

1. Procédé de fabrication d'un panier à tamis (1) pour la réception d'objets médicaux à désinfecter ou à stériliser, pour lequel à partir d'une ébauche en plaque, de préférence en tôle (2),
- une plaque de base comprenant le fond et les parois latérales du panier à tamis d'un seul tenant ou surface de base de panier à tamis (3) est fabriquée dans une première étape d'usinage (1), de préférence une séparation, le plus préférentiellement une découpe, telle qu'une découpe au laser,
- l'ébauche en plaque, en particulier en tôle (2) ou la surface de base de panier à tamis (3) est pourvue d'interruptions ou de trous (4) dans une deuxième étape d'usinage (II) se déroulant temporellement avant ou après la première étape d'usinage (I), de préférence une séparation, le plus préférentiellement un estampage afin d'obtenir une forme de départ (5) perforée,
- un plan (6) perforé ou troué est fabriqué dans une troisième étape d'usinage (III) se déroulant temporellement après la première et la deuxième étape d'usinage (I, II), de préférence un façonnage, le plus préférentiellement un laminage ou aplatissement, plan qui est divisé en une section intérieure (7) plane représentant le fond et les sections de bord (8) représentant les parois latérales,
- une forme de panier à tamis (9) avec un fond brut et des parois latérales s'étendant verticalement à celui-ci est fabriquée dans une quatrième étape d'usinage (IV) se déroulant temporellement après la troisième étape d'usinage (III), de préférence un façonnage, le plus préférentiellement un pliage, dont le fond brut (10) correspond à la section intérieure (7) plane et les parois latérales correspondent aux sections de bord (8) planes du plan perforé (6),
**caractérisé par**
une cinquième étape d'usinage (V) se déroulant temporellement directement ou indirectement après la troisième étape d'usinage (III), de préférence un façonnage, le plus préférentiellement un gaufrage qui fabrique au moins en partie au moins à partir de la section intérieure (7) plane un fond (11) structuré en trois dimensions.

2. Procédé selon la revendication 1, **caractérisé en ce que** la cinquième étape d'usinage (V) se déroule temporellement avant la quatrième étape d'usinage (IV).

3. Procédé selon la revendication 1, **caractérisé en ce que** les étapes d'usinage de la première étape d'usinage (I) à la cinquième étape d'usinage (V) se déroulent temporellement chronologiquement dans cet ordre.

4. Procédé selon l'une quelconque des revendications précédentes, **caractérisé en ce qu'** une sixième étape d'usinage, de préférence un assemblage, le plus préférentiellement un soudage, tel qu'un soudage par liaison de fusion se déroule temporellement après la quatrième étape d'usinage, qui relie entre elles les sections de bord (8) individuelles qui représentent désormais des parois latérales (13) du panier à tamis (1).

5. Procédé selon l'une quelconque des revendications précédentes, **caractérisé en ce que** les interruptions (4) obtenues dans la deuxième étape d'usinage (II) provoquent des perforations structurées différemment l'une à l'autre dans la section intérieure (7) plane et la section de bord (8).

6. Procédé selon l'une quelconque des revendications précédentes, **caractérisé en ce que** lors de la cinquième étape d'usinage (V) au moins un poinçon est utilisé, lequel est pressé par une presse sur une partie de la section intérieure (7) plane de telle manière que la partie de la section intérieure (7) s'adapte plastiquement à la forme négative du poinçon.

7. Procédé selon la revendication 6, **caractérisé en ce qu'** une pluralité de poinçons est utilisée de sorte que la section intérieure (7) plane entière soit configurée comme fond (12) structuré en trois dimensions.

8. Procédé selon l'une quelconque des revendications 6 ou 7, **caractérisé en ce que** le poinçon est tel que le fond (11) structuré en trois dimensions fabriqué dans la cinquième étape d'usinage (V) présente des ondulations ou des bosses (14) qui font saillie vers l'intérieur du panier à tamis (15) et/ou l'extérieur de panier à tamis (16) de sorte que le fond (11) présente une surface comme un treillis.

9. Procédé selon l'une quelconque des revendications 6 à 8, **caractérisé en ce que** le poinçon est tel que le fond (11) structuré en trois dimensions fabriqué dans la cinquième étape d'usinage (V) est constitué d'une pluralité de paires d'entretoises longitudinales (17) et de paires d'entretoises transversales (18) s'étendant respectivement parallèlement dans le plan de base qui s'étendent dans une vue en élévation du fond structuré (11) perpendiculairement les unes aux autres.

10. Procédé selon l'une quelconque des revendications 6 à 9, **caractérisé en ce que** le poinçon est tel que le fond (11) structuré en trois dimensions forme des surfaces d'installation et de fixation (23) pour des objets (30) à placer dans le panier à tamis (1).
